# EUROPEAN PATENT SPECIFICATION

(11) **EP 0 467 937 B1**
(45) Date of publication and mention of the grant of the patent: **28.06.1995**
(21) Application number: 90906267.1
(22) Date of filing: 10.04.1990
(51) Int. Cl.: A61B 17/32

(54) **SURGICAL BLADES**
CHIRURGISCHE KLINGEN
LAMES CHIRURGICALES

(30) Priority: 13.04.1989 GB 8908408
(43) Date of publication of application: 29.01.1992
(73) Proprietor: ETHICON LIMITED, GB-Edinburgh EH11 4HE (GB)
(72) Inventor: HOSKIN, William, John, Hertfordshire AL5 1JF (GB)
(74) Representative: Fisher, Adrian John
(86) International application number: GB9000544
(87) International publication number: WO9011724

(56) References cited:
- EP-A- 0 050 397
- EP-A- 0 119 714
- EP-A- 0 129 391
- WO-A-89/00404
- US-A- 4 387 698
- US-A- 4 411 962
- US-A- 4 647 300
- US-A- 4 702 813

## Description

The present invention relates to surgical blades.

A variety of materials have been used to provide cutting edges for surgical blades from stainless steels, to diamond.

Diamond is highly effective as a cutting edge. It has a high hardness factor and therefore can stay sharp for a long period of time. It can also be ground to define a cutting edge having a very small radius of curvature (about 15 Angstroms). It is highly resistant to corrosion. Its two main disadvantages are however its high cost and the high degree of skill and labour required to implant the cutting edge on a knife.

Stainless steel on the other hand which is polycrystalline provides a blade which is relatively cheap and easy to manufacture. Its hardness relative to diamond is very low and so is prone to rapid wear. Also it is difficult to grind the cutting edge to a radius of curvature of less than 500 Angstrom and so the cutting edge relative to diamond is blunt. Finally it is prone to corrosion, in particular where it is sterilised in a steam bath.

There has been a longfelt want in the surgical industry for a blade which has a considerably harder and sharper cutting edge than steel, is corrosion resistant and which can be produced at very much lower cost than diamond.

EP-A-0050397 discloses a razor blade having a cutting edge formed from an amorphous alloy, and a metal backing layer.

It is an object of the present invention to provide an improved surgical blade.

According to the present invention there is provided a surgical blade comprising a strip of amorphous metal with a cutting edge characterised in that the strip is flanked on each side of said cutting edge by a stiffening layer having a thickness in the range of 0.005 to 0.03 mm.

The amorphous metal is preferably an amorphous nickel or an amorphous nickel, silicon, boron alloy.

Advantageously, the strip of amorphous metal is flanked on each side by stiffening material.

The stiffening material can comprise a nickel layer electrodeposited on each side of the strip. Instead of nickel, the stiffening material can comprise electrodeposited silver. A further flash coating of gold or other material may be electrodeposited for decorative purposes.

Instead the stiffening material can comprise a sheet of material soldered or adhesively secured on opposite sides of the strip of amorphous metal.

The present invention also provides a trephine comprising a cylindrical strip of amorphous nickel or nickel alloy having a circular cutting edge, the strip when in planar form and prior to being configured into cylindrical form having been coated with a layer of nickel having a thickness in the range of 0.005 to 0.03 mm, such that when the blade is subsequently formed into a cylindrical configuration to provide the cylindrical strip, the layer of nickel acts to resist any resilience in the strip tending to return it into its planar form.

According to the present invention there is further provided a method of manufacturing a surgical blade comprising the steps of coating a strip of amorphous nickel or nickel alloy with a stiffening layer to a thickness in the range of from 0.005 to 0.030 mm, grinding the coated strip to expose the amorphous nickel or nickel alloy along one edge and to produce therein a cutting edge having a radius of curvature less than 300 Angstrom.

Surgical blades embodying the invention will now be described, by way of example, with reference to, the accompanying diagrammatic drawings in which:

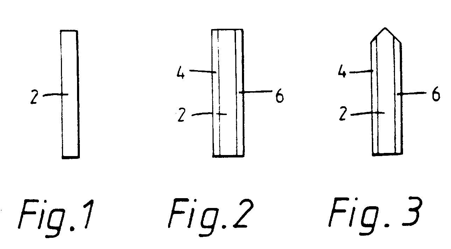

Figures 1 to 3 show three stages in the manufacture of a surgical cutting blade.

The Applicants have made a long study of different materials for use as surgical blades and by selection have discovered a particular material which has unexpected advantageous properties when used in the production of surgical blades.

A material sometimes known as Met Glas (Trade Mark) and consisting essentially of nickel in an amorphous form has been produced for the electronics industry for use as transformer laminations in areas subject to extreme environmental conditions such as may be encountered in the aerospace industry.

The material which may comprise an alloy consisting of 4-5% Silicon and 2.75 to 3.5% Boron with the balance Nickel.

To produce a surgical blade, one edge of the strip must be ground. The material was found to be extremely hard having a hardness of between 650 and 1500 Vickers but usually between 1000 to 1200 Vickers and the grinding act could provide the unexpectedly small radius of curvature of 100 Angstrom.

The material was found to be ductile in spite of its hardness with a high tensile strength. Also it was found to be highly resistant to oxidation in the presence of steam.

The sharpness of the cutting edge and the life of the blade (wear resistance) were found to be superior to steel while its cost to produce was very much less than that of diamond.

One of the main problems with a blade of this nature is that because of its thinness it is highly flexible and therefore its cutting action is difficult to control.

To ovecome this drawback the strip 2 (see Figure 1) which has a thickness of around 0.10 mm is coated on opposite sides with Nickel by electroplating to provide two layers 4 and 6 (see Figure 2) each having a thickness in the range of 0.005 to 0.030 mm. The resultant thickness of the sandwich was in the range of 0.11 mm to 0.16 mm.

A sharp cutting edge was produced by grinding through the two layers 4 and 6 along one edge and into the strip 2 to provide a cutting edge having a radius of curvature of between 100 and 300 Angstrom.

A less fine cutting edge with a radius of not less than 400 Angstrom can be provided but the preferred range for the radius is from 100 to 200 Angstrom.

Where the surgical blade is to form a trephine the strip is ground to provide a sharp cutting edge and then wrapped around a cylindrical former before being fixed in this configuration. A satisfactory trephine of 0.15 mm thickness can be produced without the need to stiffen the strip as with a rectilinear blade.

The problem is, however, that the resilience of the strip makes it difficult and therefore dangerous to handle. It could easily spring up into the face of the operator. To avoid this disadvantage the strip is electroplated with a nickel layer of from 0.05 to 0.30 mm thick and this layer has no resilience but instead suppresses the resilience of the strip. The layer of electrocoated nickel does in effect, provide some stiffening effect to the trefine in that when the coated strip is wrapped around a cylindrical former there is less tendency for the cutting edge of the blade to curl radially outwardly, as it would without the stiffening layer.

Instead of electroplating the strip with nickel it can be plated with other material, for example, silver to produce the stiffening effect.

Instead of electroplating the two stiffening plates may be bonded to the strip by means of solder or adhesive.

## Claims

1. A surgical blade comprising a strip of amorphous metal (2) with a cutting edge characterised in that the strip is flanked on each side of said cutting edge by a stiffening layer having a thickness in the range of 0.005 to 0.03 mm.

2. A surgical blade according to claim 1 wherein the amorphous metal is an amorphous nickel or an amorphous nickel, silicon, boron alloy.

3. A surgical blade according to claim 1 or claim 2 wherein the stiffening layer (4, 6) comprises a nickel layer electrodeposited on each side of the strip.

4. A surgical blade according to claim 1 or claim 2 wherein the stiffening layer (4, 6) comprises a sheet of material soldered or adhesively secured on opposite sides of the strip of amorphous metal (2).

5. A surgical blade according to claim 1 or claim 2 wherein the stiffening layer (4, 6) comprises electrodeposited silver.

6. A trephine comprising a cylindrical strip of amorphous nickel or nickel alloy having a circular cutting edge, the strip when in planar form and prior to being configured into cylindrical form having been coated with a layer of nickel having a thickness in the range of 0.005 to 0.03 mm, such that when the blade is subsequently formed into a cylindrical configuration to provide the cylindrical strip, the layer of nickel acts to resist any resilience in the strip tending to return it into its planar form.

7. A blade or trephine according to any preceding claim including a flash coating of gold or other material electrodeposited for decorative purposes.

8. A blade or trephine according to any preceding claim wherein the cutting edge has a radius of curvature of less than 300 Angstrom.

9. A method of manufacturing a surgical blade comprising the steps of coating a strip (2) of amorphous nickel or nickel alloy with a stiffening layer (4, 6) to a thickness in the range of from 0.005 to 0.030 mm, grinding the coated strip to expose the amorphous nickel or nickel alloy along one edge and to produce therein a cutting edge having a radius of curvature less than 300 Angstrom.

## Patentansprüche

1. Chirurgische Klinge mit einem Streifen aus amorphem Metall (2) mit einer Schneidkante, dadurch gekennzeichnet, daß der Streifen auf jeder Seite der Schneidkante von einer Versteifungsschicht mit einer Dicke im Bereich von 0,005 bis 0,03 mm begrenzt wird.

2. Chirurgische Klinge nach Anspruch 1, wobei das amorphe Metall amorphes Nickel oder eine Legierung aus amorphem Nickel, Silizium und Bor ist.

3. Chirurgische Klinge nach Anspruch 1 oder 2, wobei die Versteifungsschicht (4, 6) eine Nickelschicht aufweist, die durch galvanischen Niederschlag auf jeder Seite des Streifens aufgebracht ist.

4. Chirurgische Klinge nach Anspruch 1 oder 2, wobei die Versteifungsschicht (4, 6) ein dünnes Blech aufweist, das auf beide Seiten des Streifens aus amorphem Metall (2) gelötet oder mit diesem klebend verbunden ist.

5. Chirurgische Klinge nach Anspruch 1 oder 2, wobei die Versteifungsschicht (4, 6) durch galvanischen Niederschlag aufgebrachtes Silber aufweist.

6. Trephine mit einem zylindrischen Streifen aus amorphem Nickel oder aus einer Nickellegierung mit einer kreisförmigen Schneidkante, wobei der Streifen, als er eine ebene Form aufwies und bevor er in zylindrische Form gebracht wurde, mit einer Nickelschicht einer Dicke im Bereich von 0,005 bis 0,03 mm beschichtet wurde, wobei die Nickelschicht bei einer nachfolgenden Verformung der Klinge in eine zylindrische Konfiguration zur Herstellung des zylindrischen Streifens dazu dient, einer Rückfederung des Streifens in seine ebene Form entgegenzuwirken.

7. Klinge oder Trephine nach einem der vorangehenden Ansprüche, die für dekorative Zwecke mit einem durch galvanischen Niederschlag aufgebrachten Glanzüberzug aus Gold oder anderem Material versehen ist.

8. Klinge oder Trephine nach einem der vorangehenden Ansprüche, wobei die Schneidkante einen Krümmungsradius von weniger als 300 Angström aufweist.

9. Verfahren zur Herstellung einer chirurgischen Klinge mit den Verfahrensschritten, daß ein Streifen (2) amorphen Nickels oder einer Nickellegierung mit einer Versteifungsschicht (4, 6) mit einer Dicke im Bereich von 0,005 bis 0,03 mm beschichtet, daß der beschichtete Streifen zum Freilegen des amorphen Nickels oder der Nickellegierung entlang einer Kante geschliffen, und daß darin eine Schneidkante mit einem Krümmungsradius von weniger als 300 Angström ausgebildet wird.

## Revendications

1. Lame chirurgicale constituée d'une bande de métal amorphe (2) avec un bord de coupe caractérisé en se que la bande est bordée sur chaque côté dudit bord de coupe par une couche de renfort ayant une épaisseur dans la fourchette de 0,005 à 0,03 mm.

2. Lame chirurgicale selon la revendication 1, dans laquelle le métal amorphe est un nickel amorphe ou un alliage amorphe de nickel, de silicium et de bore.

3. Lame chirurgicale selon la revendication 1 ou la revendication 2, dans laquelle la couche de renfort (4, 6) est comstituée d'une couche de nickel déposée par électrolyse sur chaque côté de la bande.

4. Lame chirurgicale selon la revendication 1 ou la revendication 2, dans laquelle la souche de renfort (4, 6) est constituée d'une feuille de matériau soudée ou fixée de façon adhésive sur des côtés opposés de la bande de métal amorphe (2).

5. Lame chirurgicale selon la revendication 1 ou la revendication 2, dans laquelle la couche de renfort (4, 6) est constituée d'argent déposé par électrolyse.

6. Trépan constitué d'une bande cylindrique de nickel amorphe ou d'alliage de nickel, ayant un bord de soupe circulaire, la bande, lorsqu'elle est de forme plane et avant d'être configurée sous forme de cylindre ayant été revétue d'une couche de nickel ayant une épaisseur dans la fourchette de 0,005 a 0,03 mm, de sorte que lorsque la lame est formée, par la suite, en une configuration cylindrique, pour créer une bande cylindrique, la couche de nickel agit pour résister a n'importe quelle élasticité dans la bande tendant à la faire retourner a sa forme plane.

7. Lame ou trépan selon l'une quelconque des revendications précédentes comprenant un revêtement d'un voile d'or ou d'une autre matière déposé par électrolyse à des fins décoratives.

8. Lame ou trépan selon l'une quelconque des revendications précédentes dans lequel le bord de coupe a un rayon de courbure de moins de 300 Angstrom.

9. Procédé de fabrication d'une lame chirurgicale comprenant les phases de revêtement d'une bande (2) de nickel amorphe ou d'alliage de nickel, avec une couche de renfort (4, 6) sur une épaisseur dans la fourchette de 0,005 à 0,030 mm, de rectification de la bande revêtue pour mettre à nu le nickel amorphe ou l'alliage de nickel le long d'un bord et pour réaliser dedans un bord de coupe ayant un rayon de courbure inférieur à 300 Angstrom.
